# EUROPÄISCHE PATENTSCHRIFT

(11) **EP 3 493 891 B1**
(45) Veröffentlichungstag und Bekanntmachung des Hinweises auf die Patenterteilung: **25.12.2019**
(21) Anmeldenummer: 17712945.9
(22) Anmeldetag: 20.03.2017
(51) Int. Cl.: B01D 46/00, B01D 53/88, B60H 3/06

(54) **LUFTREINIGUNGSVORRICHTUNG**
AIR PURIFICATION DEVICE
DISPOSITIF D'ÉPURATION DE L'AIR

(30) Priorität: 21.09.2016 DE 102016117797; 28.10.2016 DE 102016120656
(43) Veröffentlichungstag der Anmeldung: 12.06.2019
(73) Patentinhaber: Dr. Schneider Kunststoffwerke GmbH, 96317 Kronach (DE)
(72) Erfinder: GRÜNBECK, Thomas, 96358 Teuschnitz (DE); ENDRES, Gerhard, 95336 Mainleus (DE); FIEDLER, Jochen, Pressig 96332 (DE)
(86) Internationale Anmeldenummer: PCT/EP2017/056523
(87) Internationale Veröffentlichungsnummer: WO 2018/054555

(56) Entgegenhaltungen:
- WO-A1-2005/108137
- FR-A1- 2 838 379
- JP-A- H09 309 329
- JP-A- 2004 305 436
- JP-B1- 3 390 429

## Beschreibung

Die vorliegende Einrichtung dient zur Reinigung von Luft, insbesondere zur Reinigung von Luft in Kraftfahrzeuginnenräumen.

Aus dem Stand der Technik sind verschiedene Vorrichtungen zur Luftreinigung in Kraftfahrzeugen bekannt. Diese sind zumeist derart ausgebildet und angeordnet, dass Luft kontinuierlich aus dem Fahrzeuginnenraum abgesaugt, über einen Filter geführt und dann dem Fahrzeuginnenraum wieder zugeführt wird.

So ist aus der DE 10 2014 012 870 A1 ein Luftreiniger bekannt. Dieser Luftreiniger verwendet ultraviolette Strahlen zur Luftreinigung. Der Luftreiniger weist ein Gehäuse mit einem Gehäuseeinlass und einem -auslass, ein Gebläse, das im inneren des Gehäuses angrenzend an den Lufteinlass angeordnet ist, eine ultraviolette Leuchtdiodeneinheit und eine Filtereinheit auf. Die Filtereinheit ist im Inneren des Gehäuses über dem Gebläse entlang eines Strömungsweges der Luft angeordnet. Es ist außerdem eine Strömungsanordnung vorhanden, die im Inneren des Gehäuses zwischen dem Gebläse und der Filtereinheit angeordnet ist. Dabei steuert die Strömungssteueranordnung den Luftstrom entlang des Strömungswegs der Luft zwischen dem Auslass des Gebläses und der Filtereinheit. Zur Reinigung der Luft kommen hierbei fotokatalytische, ultraviolette Leuchtdioden zum Einsatz.

Aus der DE 20 2007 019 288 U1 ist ein System zur Luftreinigung unter Verwendung von Ozon und einem keramischen, porösen Katalysator offenbart. Das System weist ein Gehäuse, mindestens einen Einlass und einen Auslass, mindestens eine Photonenquelle, einen keramischen Kern und ein Fluidstromerzeugungsgerät auf, wobei die Photonenquelle stromaufwärts von dem keramischen Kern angeordnet ist.

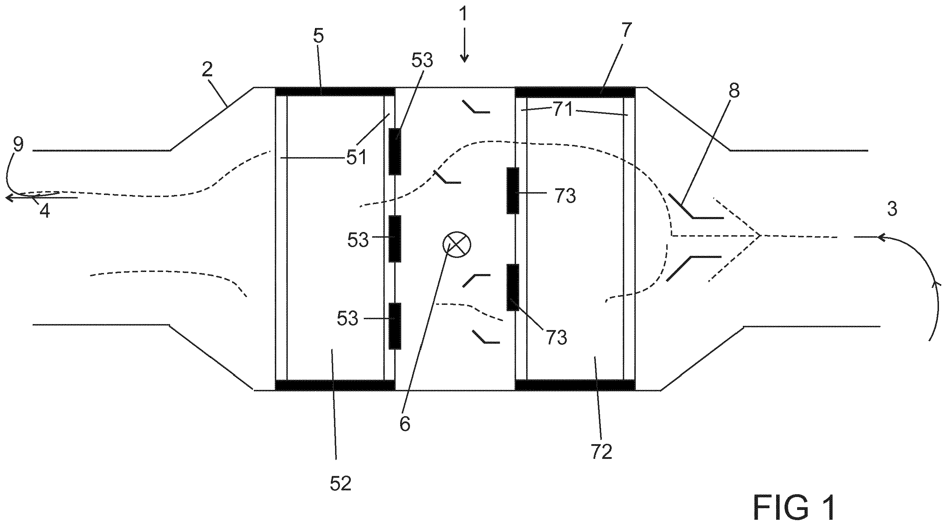

Aus der FR 2 838 379 A1 ist eine erste Luftreinigungsvorrichtung beschrieben. Diese besitzt eine titanoxidhaltige Platte und eine zugeordnete UV-Lampe. Die Luftreinigungsvorrichtung ist im Bereich des Verdampfers einer Klimaanlage angeordnet. Durch die photokatalytische Wirkung der titanoxidhaltigen Platte bei Bestrahlung mit der UV-Lampe wird die vorbeiströmende Luft gereinigt. Dies ergibt sich beispielhaft anhand der Beschreibung der Figuren 1 bis 8. Es ist dortig der Bereich des Verdampfers offenbart und in den Verdampfer bzw. am Verdampfer sind entsprechend Titanschichten eingearbeitet. Hier ist kein Vlies verwendet, es kommt auch kein Aktivkohlebereich zur Anwendung. Es ist aber auch eine zweite Luftreinigungsvorrichtung offenbart, welche im Bereich eines Luftausströmers in einem Kraftfahrzeug angeordnet ist. Diese Luftreinigungsvorrichtung besitzt photokatalysatorische Mittel, die mit Körnern eines Absorbtionsmittels verbunden sind. Das Absorbtionsmittel absorbiert Schadstoffe in seinen Poren, fängt diese ein ohne sie zu zerstören. Anschließend werden über die Photokatalyse die aufgefangenen Schadstoffe durch den Photokatalysationsprozess zerstört.

Aus der EP 0 707 989 A1 ist eine Vorrichtung zur Reinigung von Luft bekannt. Eine Reinigung erfolgt in der Weise, dass Außenluft über ein Gehäuse und ein darin angeordnetes Gebläse angesaugt, in mehreren Reinigungsstufen gereinigt und dann dem Innenraum des Fahrzeugs zugeführt wird. Das Gebläse bzw. der Antrieb des Gebläses wird über Solarzellen, die am Fahrzeug angeordnet sind und als Energiequellen dienen, gespeist.

Nachteilig bei dem vorgenannten Stand der Technik ist, dass die Reinigungswirkung und insbesondere die Beseitigung von Schadpartikeln oder Geruchspartikeln aus der zu reinigenden Luft nicht ausreichend und nicht über einen längeren Zeitraum ohne Wartungseingriffe möglich sind. Dies ist zum einen dadurch bedingt, dass zumeist ein mechanischer Filter verwendet wird, der nach relativ kurzer Zeit bereits eine solche Menge an Partikeln aufgenommen hat, dass er getauscht werden muss, um die ursprüngliche Reinigungsleistung beizubehalten. Erfolgt kein regelmäßiger Austausch, trägt ein solcher Filter mehr zur Verunreinigung der Luft bei, als dass er diese reinigt. Es dürfte den meisten Benutzern von Klimaanlagen bekannt sein, dass ein verschmutzter Filter erheblich zur Geruchsbelästigung in einem Kraftfahrzeug beiträgt.

Weiterhin ist nachteilig, dass ein Filterwechsel mit relativ hohen Kosten verbunden ist, da ein solcher Wechsel zumeist nur in einer Fachwerkstatt vorgenommen werden kann.

Im Weiteren ist zu berücksichtigen, dass vor allem neue Fahrzeuge über einen bestimmten Zeitraum flüchtige organische Verbindungen (VOC) und andere gesundheitsschädlich Substanzen abgeben. Um diese möglichst umgehend und ohne Belastung für die Insassen eines Fahrzeugs zu beseitigen, sind aus dem Stand der Technik bisher keine Systeme bekannt. Zwar sind im Stand der Technik Filtersysteme, wie eingangs beschrieben, vorhanden, welche auch solche Verbindungen herausfiltern können, jedoch ist der Zeitraum, über welchen sich diese Verbindung im Fahrzeug halten, verhältnismäßig lang.

Es ist daher Aufgabe der vorliegenden Erfindung, die Nachteile des Standes der Technik zu beheben und eine Luftreinigungsvorrichtung anzugeben, welche flüchtige organische Stoffe und gesundheitsschädlich Substanzen sowie andere Luftverunreinigungen gezielt in der Luft im Fahrzeuginnenraum reduziert und zusätzlich auch unangenehme Gerüche vermeidet und beseitigt.

Diese Aufgabe wird durch eine Luftreinigungsvorrichtung mit den in Anspruch 1 angegebenen technischen Merkmalen gelöst. Vorteilhafte Weiterbildungen sind in den Unteransprüchen, der weiteren Beschreibung und insbesondere der Beschreibung anhand konkreter Ausführungsbeispiele, angegeben.

Die vorliegende Erfindung betrifft eine Luftreinigungseinheit mit einem Gehäuse, das mindestens eine Eingangsöffnung zur Zuführung eines Luftstroms und mindestens eine Ausgangsöffnung zur Ausleitung des über die Eingangsöffnung zugeführten Luftstroms besitzt. Im Gehäuse sind mindestens eine Luftreinigungseinheit und mindestens eine Beleuchtungseinheit angeordnet. Die mindestens eine Luftreinigungseinheit nimmt im Wesentlichen die Form des Querschnitts des Gehäuses auf und die Querschnittsfläche ist nahezu ausfüllend im Gehäuse angeordnet. Im Gehäuse ist die mindestens eine Beleuchtungseinheit der mindestens einen Luftreinigungseinheit gegenüberliegend angeordnet. Die mindestens eine Luftreinigungseinheit ist luftdurchlässig ausgestaltet und weist auf der der Beleuchtungseinheit zugwandten Seite mindestens einen photokatalytisch wirksamen Oberflächenbereich bzw. Flächenbereich auf. Außerdem weist sie mindestens einen mit Aktivkohle durchsetzten bzw. versetzten Bereich, der zumindest partiell von einem Vlies umgeben ist, auf, wobei auf der der Beleuchtungseinheit zugwandten Seite der mindestens einen Luftreinigungseinheit mindestens einen photokatalytisch wirksamen Oberflächenbereich im oder am Vlies vorhanden ist und der Luftstrom im Gehäuse zumindest partiell den mindestens einen photokatalytisch wirksamen Oberflächenbereich und den mit Aktivkohle versetzen Flächenbereich umströmt bzw. durchströmt. Der mindestens eine photokatalytisch wirksame Oberflächenbereich der mindestens einen Luftreinigungseinheit ist von der mindestens einen Beleuchtungseinheit mit Licht anstrahlbar.

In einer vorteilhaften Ausgestaltung ist auf der der Beleuchtungseinheit abgewandte Seite mindestens ein mit Aktivkohle versetzter bzw. durchsetzter Flächenbereich vorhanden. Der Luftstrom im Gehäuse durchströmt dann zumindest partiell den mindestens einen photokatalytisch wirksamen Oberflächenbereich und den mit Aktivkohle versetzten Flächenbereich.

In einer weiteren vorteilhaften Ausgestaltung der Erfindung ist der auch das Vlies mit Aktivkohle versetzt bzw. durchsetzt.

In einer vorteilhaften Ausgestaltung der Erfindung nach Patentanspruch 2 ist vorgesehen, dass der mindestens eine photokatalytisch wirksame Oberflächenbereich zumindest teilweise mit einem Metalloxid oder einem Mischoxid beschichtet oder mit einem Metalloxid oder einem Mischoxid - versetzt oder durchsetzt ist.

Unter einem Mischoxid, kurz auch mit MOX bzw. MOx bezeichnet, versteht man einen Stoff, der sich aus mehreren Oxiden zusammensetzt. Es gibt eine Vielzahl von Mischoxiden. Unter einem Metalloxid versteht man eine Verbindung zwischen einem Metall und Sauerstoff.

In einer vorteilhaften Ausgestaltung der Erfindung nach Patentanspruch 3 ist vorgesehen, dass das Metalloxid ein Oxid aus einem Übergangsmetall ist.
Als Übergangselemente werden die chemischen Elemente mit den Ordnungszahlen von 21 bis 30, 39 bis 48, 57 bis 80 und 89 bis 112 bezeichnet. Da diese Elemente alle Metalle sind, werden diese Elemente auch als Übergangsmetalle bezeichnet. Dieser Begriff Übergangsmetalle ergibt sich auf Grund der Position dieser Elemente im Periodensystem begründet, da sich dort der Übergang durch die aufeinanderfolgende Zunahme von Elektronen in den d-Atomorbitalen entlang jeder Periode zeigt. Übergangselemente werden von der IUPAC definiert als Elemente, die eine unvollständige d-Unterschale besitzen oder Ionen mit einer unvollständigen d-Unterschale ausbilden.

In einer vorteilhaften Ausgestaltung der Erfindung nach Patentanspruch 4 ist vorgesehen, dass das Metalloxid oder Mischoxid von aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃, LiNbO₃ und/oder Nb₂O₅ besteht.

Bei photochemischen Katalysatoren, wie im vorliegenden Fall, können einzelne Oxide von Übergangsmetallen in Form von Mischoxiden zum Einsatz kommen. Diese Katalysatoren mit Oxiden von Übergangsmetallen sind wirksam und erwirken eine vollständige und/oder selektive Oxidation von flüchtigen organischen Verbindungen.

Die katalytische Oxidation von flüchtigen organischen Verbindungen mittels Katalysatoren auf Basis von Edelmetallen ist für deren Struktur und deren strukturellen Eigenschaften teilweise schädlich. Die Wirkung von Pt-Partikelgröße (Pt = Platin) auf die katalytische Oxidation von verschiedenen Kohlenwasserstoffen hat gezeigt, dass im Allgemeinen größere Pt-Teilchen aktiver sind als kleinere PT-Teilchen. Eine geringere Auswirkungen auf die katalytische Wirksamkeit von Pt-Katalysatoren ist durch Faktoren wie die Art des Trägers (Aluminiumoxid oder Siliciumdioxid) ausgeübt wird, die Porosität und Säure-Base-Eigenschaften des Trägers. Die Zugabe von Co₃O₄, CeO₂, La³⁺ / Bi³⁺ Promotoren zu CeO₂-ZrO₂ führt zu einer Erhöhung der Aktivität und Wärmestabilität von Katalysatoren mit Pt und Pd (Palladium) auf Basis von Aluminiumoxid, sowie zur Reduktion von flüchtigen organischen Verbindungen.
Edelmetall-Katalysatoren, wie Pt und Pd (Palladium), zeigen eine gute Wirkung bei niedrigen Temperaturen in vollständige Oxidation von flüchtigen organischen Verbindungen. Die Anwendung derartiger Katalysatoren zur Katalyse von flüchtigen organischen Verbindungen ist zum Einsatz im Innenraum von Fahrzeugen begrenzten aufgrund der hohen Kosten und der technisch bedingten Gefahr von Vergiftungen auf Grund der möglichen Bildung von Chlor und Chlorderivaten. Ceriumoxid hingegen ist sehr aktiv auf Grund seiner Fähigkeit von Sauerstoffanreicherung. Oxidierung von flüchtigen organischen Verbindungen zu CeO₂ basiert auf dem Redox-Mechanismus. Die Modifikation von CeO₂ mit anderen Metalloxiden, z.B. durch teilweisen Ersatz von Ce⁴⁺-Ionen durch Zr⁴⁺-Ionen im Gitternetz (gemischte Ce-Zr-Oxide), kann die Sauerstoffkapazität und thermische Beständigkeit des Katalysators, sowie Erhöhung der katalytischen Aktivität bei niedrigen Temperaturen, verbessern. Ein Vorteil der Katalysatoren auf Manganbasis ist deren hohe Aktivität für alle Oxidationsreaktionen im Zusammenhang mit damit entstehenden niedrigen Kosten und geringen Toxizität.
Auch Katalysatoren mit der Perowskit-Struktur können zum Einsatz kommen.
Sehr gute katalytische Eigenschaften werden auch von Perowskit-Strukturen mit deren allgemeine Formel ABO₃ erzielt. Bei dieser Formel ist A das Element der Seltenen Erden, B ein Übergangsmetall ist.

In einer besonders gut zu realisierenden Ausgestaltung der Erfindung ist der mindestens eine photokatalytisch wirksame Oberflächenbereich zumindest teilweise mit Titandioxid - TiO2 - beschichtet oder mit Titandioxidionen - TiO2-Ionen - versetzt oder durchsetzt. Es kann aber auch eine jeder andere der vorgenannten Verbindungen, die photokatalytisch wirksam ist, zum Einsatz kommen.

In einer vorteilhaften Ausgestaltung der Erfindung nach Patentanspruch 5 ist vorgesehen, dass die mindestens eine Luftreinigungseinheit an einer der Innenwände des Gehäuses angeordnet oder fixierbar ist. Durch die Anordnung bzw. Fixierung der mindestens einen Luftreinigungseinheit an einer der Innenwände des Gehäuses kann eine gute Befestigung erfolgen, zugleich kann der Luftstrom im Gehäuse so gelenkt werden, dass er optimal auf die mindestens eine Luftreinigungseinheit abgestimmt ist. Bei einer Anordnung in der Mitte des Gehäuses kann die mindestens eine Luftreinigungseinheit direkt vom in das Gehäuse einströmenden Luftstrom umgeben werden und es wird eine gute Reinigungsleistung erzielt.

In einer vorteilhaften Ausgestaltung der Erfindung nach Patentanspruch 6 ist vorgesehen, dass die mindestens eine Beleuchtungseinheit im Gehäuse der mindestens einen Luftreinigungseinheit gegenüberliegend zum mindestens einen photokatalytisch wirksamen Oberflächenbereich der mindestens einen Luftreinigungseinheit auf einer der Innenwände des Gehäuses oder nahezu in der Mitte des Gehäuses angeordnet ist. Die Innenwände des Gehäuses sind im Bereich zwischen der mindestens einen Luftreinigungseinheit und der mindestens eine Beleuchtungseinheit mit einem lichtreflektierenden Material, insbesondere einem UV-Licht reflektierenden Material, beschichtet.
Die Beleuchtungseinheit ist zwingend notwendig, da nur durch auf die mindestens eine Luftreinigungseinheit bzw. deren photokatalytisch wirksame Oberflächenbereichen auftreffendes Licht, vorzugsweise UV-Licht, der photokatalytische Reinigungsprozess in Gang setzt. Daher ist durch die Anordnung der mindestens einen Beleuchtungseinheit gegenüber der mindestens einen Luftreinigungseinheit eine gute Beleuchtung und damit Zuführung von UV-Licht möglich.

In einer vorteilhaften Ausgestaltung der Erfindung ist vorgesehen, dass die mindestens eine Beleuchtungseinheit aus mindestens einer Leuchtdiode besteht, die UV-Licht emittiert. Je nach Dotierung des Photokatalysators kann eine Aktivierung auch mit sichtbarem Licht erfolgen.

In einer vorteilhaften Ausgestaltung der Erfindung nach Patentanspruch 7 ist vorgesehen, dass vor der mindestens einen Beleuchtungseinheit Linsen angeordnet sind, die das von der mindestens einen Beleuchtungseinheit emittierte Licht auf die mindestens einen Luftreinigungseinheit fokussieren oder hinter und/oder neben der mindestens einen Beleuchtungseinheit Lichtreflektoren oder lichtreflektierende Bereiche angeordnet sind, welche das von der mindestens einen Beleuchtungseinheit emittierte Licht in Richtung der mindestens einen Luftreinigungseinheit reflektieren. Durch die Anordnung der Linsen wird eine besonders gute Fokussierung des emittierten Lichts auf die mindestens einen Luftreinigungseinheit erreicht. Durch die Anordnung von Lichtreflektoren oder lichtreflektierende Bereiche wird erreicht, dass auch Licht, das nicht direkt auf die mindestens eine Luftreinigungseinheit gerichtet wird, dennoch zumindest teilweise zur Luftreinigungseinheit geleitet wird.

In einer vorteilhaften Ausgestaltung der Erfindung nach Patentanspruch 8 ist vorgesehen, dass die Innenwände des Gehäuses weiß lackiert oder mit einem lichtreflektierenden Material beschichtet sind. Dadurch wird erreicht, dass das von der mindestens einen Beleuchtungseinheit emittierte Licht nicht vom Gehäuse absorbiert wird, sondern reflektiert und damit zusätzlich auf die bzw. in Richtung der mindestens einen Luftreinigungseinheit geleitet wird.

In einer vorteilhaften Ausgestaltung der Erfindung nach Patentanspruch 9 ist vorgesehen, dass im Gehäuse Luftleitelemente angeordnet sind, welche den Luftstrom diffus im Gehäuse ablenken, sodass der Luftstrom nahezu gleichmäßig auf die gesamte Oberfläche des Vlieses der mindestens einen Luftreinigungseinheit trifft und diese durchströmt oder der Luftstrom größtenteils auf den mindestens einen photokatalytisch wirksamen Oberflächenbereich gerichtet ist und diesen umströmt bzw. durchströmt. Zusätzlich entstehen dadurch gewollte Verwirbelungen der Luft. Damit wird eine besonders hohe Reinigungsleistung erzielt.

In einer vorteilhaften Ausgestaltung der Erfindung nach Patentanspruch 10 ist vorgesehen, dass die mindestens eine Luftreinigungseinheit aus einem Rahmen aus Kunststoff oder Metall besteht, welcher vom Vlies zumindest an einer Seite umschlossen ist und hinter dem Vlies ein Aufnahmebereich für Granulatstoffe vorhanden ist, wobei dieser Aufnahmebereich mit Aktivkohle oder einem Gemisch aus Aktivkohle und/oder mit Titandioxid - TiO2 - beschichtet und/oder mit Titandioxidionen - TiO2-Ionen - versetztem Granulat befüllt ist.
Damit kann durch den photokatalytischen Prozess, der bei lichtauftreffen auf Titandioxid ausgelöst wird, auch der Aktivkohlebereich gereinigt werden, sodass der Filter längere Zeit seine vollständige Reinigungsleistung erreicht. Außerdem können dadurch Verunreinigungen, die am Vlies anhaften, entfernt bzw. beseitigt werden.

Es ist vorgesehen, dass zumindest partiell Fäden oder Fasern in das Vlies eingearbeitet oder eingebracht sind, die mit Titandioxidionen - TiO2-Ionen dotiert sind, welche den mindestens einen photokatalytisch wirksamen Oberflächenbereich ausbildet oder dass Bereiche des Vlieses mit mit Titandioxidionen - TiO2-Ionen dotiert sind. Es wird durch den photokatalytischen Prozess, der bei lichtauftreffen auf Titandioxid aktiviert wird, nicht nur die vorbeiströmende Luft gereinigt, sondern zugleich auch der Aktivkohlebereich und am Vlies anhaftende Verunreinigungen werden entfernt, sodass der Filter längere Zeit seine vollständige Reinigungsleistung erreicht.

In einer vorteilhaften Ausgestaltung der Erfindung nach Patentanspruch 11 ist vorgesehen, dass die Oberfläche der mindestens einen Luftreinigungseinheit mit dem Vlies zumindest an der den mindestens einen photokatalytisch wirksamen Oberflächenbereich aufweisenden Seite wellenförmig ausgestaltet ist oder kegel-, falten-, zylinder-, kegelstumpf-, pyramidenstumpf-, kugel- oder halbkugelgeometrische Ausformungen besitzt. Durch diese Formgebung kann die für die Luftreinigung wirksame Oberfläche der mindestens einen Luftreinigungseinheit vergrößert werden. Die konkrete Formgestaltung der Oberfläche führt nur zu einer geringen Erhöhung des Luftwiderstandes, der dem Luftstrom im Gehäuse entgegenwirkt.

In einer vorteilhaften Ausgestaltung der Erfindung nach Patentanspruch 12 ist vorgesehen, die mindestens eine Beleuchtungseinheit in ihrer Lichtintensität steuerbar ist, wobei ein Luftgütemesssensor, der im Lufteinlasskanal oder am Luftauslasskanal angeordnet ist, die Luftgüte ermittelt und eine Steuereinheit anhand der vom Luftgütemesssensor ermittelten Luftgüte die Lichtintensität der mindestens einen Beleuchtungseinheit steuert. Damit kann zum einen die elektrische Leistungsaufnahme bedarfsgerecht gesteuert werden, zugleich kann vermieden werden, dass die mindestens eine Beleuchtungseinheit kontinuierlich, auch wenn nicht erforderlich, mit maximaler elektrischer Last, betrieben wird. Dies ermöglicht zugleich eine Verlängerung der Lebensdauer der mindestens einen Beleuchtungseinheit.

In einer vorteilhaften Ausgestaltung der Erfindung nach Patentanspruch 13 ist vorgesehen, dass die mindestens eine Beleuchtungseinheit in ihrer Lichtintensität von der Steuereinheit derart steuerbar ist, dass die Steuereinheit die Lichtintensität der mindestens einen Beleuchtungseinheit in Abhängigkeit der Drehzahl eines Ventilators steuert, der vor dem Lufteinlasskanal oder dem Luftauslasskanal angeordnet ist. Es kann somit zum einen die elektrische Leistungsaufnahme bedarfsgerecht gesteuert werden, zugleich kann vermieden werden, dass die mindestens eine Beleuchtungseinheit kontinuierlich, auch wenn nicht erforderlich, mit maximaler elektrischer Last, betrieben wird. Die mindestens eine Beleuchtungseinheit wird in ihrer Intensität in Abhängigkeit der das Gehäuse durchströmenden Luftmenge gesteuert, somit erfolgt eine bedarfsgerechte Ansteuerung. Dies ermöglicht außerdem eine Verlängerung der Lebensdauer der mindestens einen Beleuchtungseinheit, da diese nicht stets mit maximaler Last betrieben wird.

In einer vorteilhaften Ausgestaltung der Erfindung nach ist vorgesehen, dass die Luftreinigungseinheit im oder beim Lufteinzug der Umluftklappe eines Kraftfahrzeuges angeordnet ist. Es wird in diesem Fall kein eigener Lüfter für die Luftreinigungseinheit benötigt, da der Luftfluss des Umluftbetriebs ausgenutzt und mitgenutzt werden kann.

In einer vorteilhaften Ausgestaltung der Erfindung nach Patentanspruch 14 ist vorgesehen, dass ein VOC Sensor zur bedarfsgerechten Steuerung der Luftreinigungsvorrichtung in dieser angeordnet ist oder dass ein im Kraftfahrzeug bereits vorhandenen CO2-Sensor zur bedarfsgerechten Steuerung der Luftreinigungsvorrichtung eingebunden ist.

Im Folgenden wird die erfindungsgemäße Luftreinigungsvorrichtung anhand konkreter Ausführungsbeispiele anhand von Figuren beschrieben. Die nachfolgende Beschreibung anhand der konkreten Ausführungsbeispiele stellt keine Limitierung der Erfindung auf eines dieser konkreten Ausführungsbeispiele dar.

In den Figuren zeigt:
- FIG 1: einen schematischen Aufbau einer erfindungsgemäßen Luftreinigungsvorrichtung;
- FIG 2: eine perspektivische Ansicht einer Luftreinigungsvorrichtung und
- FIG 3: eine Ansicht einer erfindungsgemäßen Luftreinigungsvorrichtung mit den weiteren relevanten Bestandteilen;

In den Figuren sind gleiche Teile und/oder Komponenten mit gleichen Bezugszeichen versehen. Diese Teile und/oder Komponenten entsprechen im Wesentlichen einander, solange nichts anderen angegeben ist.

In FIG 1 ist ein Schnitt durch eine schematische Luftreinigungsvorrichtung 1 dargestellt. Die Luftreinigungsvorrichtung 1 besitzt ein Gehäuse 2. Die Form des Gehäuses 2 kann einen runden, ovalen, sechseckigen, n-eckigen oder, vorzugsweise, einen rechteckigen Querschnitt besitzen.

Als besonders vorteilhaft hat sich die Ausgestaltung des Querschnitts des Gehäuses 2 in einer rechteckigen Form ergeben. Das Gehäuse 2 ist quaderförmig.

Das Gehäuse 2 besteht vorzugsweise aus Kunststoff; in einer besonderen Ausgestaltung ist der gewählte Kunststoff ABS.

Das Gehäuse 2 weist an zwei sich gegenüberliegenden Enden eine Einlassöffnung in Form eines Lufteinlasskanals 3 und eine Auslassöffnung in Form eines Luftauslasskanals 4 für über den Lufteinlasskanal 3 dem Gehäuse 2 zugeführte Luft, auf.

Über den Lufteinlasskanal 3 wird zu reinigende Luft der Luftreinigungsvorrichtung 1 in deren Gehäuse 2 zugeführt; über den Luftauslasskanal 4 wird die in der Luftreinigungsvorrichtung 1 gereinigte Luft wieder aus dem Gehäuse 2 herausgeleitet.

Im Gehäuse 2 bildet sich ein Luftstrom 9. Der Luftstrom 9 durchläuft das Gehäuse 2 vom Lufteinlasskanal 3 zum Luftauslasskanal 4. Der Luftstrom ist im Gehäuse 2 gerichtet.

Im Gehäuse 2 sind zwei Luftreinigungseinheiten 5, 7 angeordnet. Es können aber auch weitere Luftreinigungseinheiten im Gehäuse 2 angeordnet sein, oder aber nur eine einzige Luftreinigungseinheit 5.

Die Luftreinigungseinheiten 5, 7 sind an einer der Innenwände des Gehäuses 2 angeordnet und mit der Innenwand form- oder kraftschlüssig verbindbar. Hierzu sind Klipse oder Halter vorgesehen, welche die Luftreinigungseinheiten 5, 7 an der Innenwand des Gehäuses 2 fixieren.

Zwischen den beiden Luftreinigungseinheiten 5, 7 ist eine Beleuchtungseinheit 6 angeordnet. Es können auch mehrere Beleuchtungseinheiten 6 im Gehäuse 2 angeordnet werden.

Die Beleuchtungseinheit 6 ist nahezu mittig im Gehäuse 2 oder der Innenwand des Gehäuses 2 so angeordnet, dass das von der Beleuchtungseinheit 6 emittierte Licht nahezu vollständig auf die Luftreinigungseinheit 5 und die Luftreinigungseinheit 7 gerichtet abgegeben wird.

In einer vorteilhaften Ausgestaltung der Erfindung sind vor der Beleuchtungseinheit 6 Linsen angeordnet, die das Licht der Beleuchtungseinheit 6 auf die Luftreinigungseinheiten 5, 7 fokussieren.

In einer weiteren Ausgestaltung der Erfindung sind Prismen oder Spiegel an der Beleuchtungseinheit 6 angeordnet, welche verhindern, dass das abgegebene Licht der Beleuchtungseinheit 6 nicht auf die Luftreinigungseinheiten 5, 7 trifft.

In einer weiteren vorteilhaften Ausgestaltung der Erfindung ist das Innere des Gehäuses 2 weiß lackiert oder mit einer lichtreflektierenden Oberflächenbeschichtung versehen.

Die Luftreinigungseinheiten 5, 7 besitzen jeweils mindestens einen photokatalytisch wirksamen Bereich 53, 73. In der Ausgestaltung nach FIG 1 sind bei der Luftreinigungseinheiten 5 drei derartige Bereiche 53 vorhanden, bei der Luftreinigungseinheiten 7 sind es zwei derartige Bereiche 73.

Die photokatalytisch wirksamen Bereiche 53, 73 bestehen in der konkreten Ausgestaltung nach FIG 1 aus Titandioxid - TiO2 - oder sind mit Titanoxidionen - TiO2-Ionen - dotiert oder versetzt oder durchsetzt. Es können jedoch auch andere photokatalytisch aktive Materialien als TiO2 eingesetzt werden, insbesondere die bereits genannten Materialien, insbesondere Metalloxid oder Mischoxid von aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃, LiNbO₃ und/oder Nb₂O₅. Es können aber auch andere photokatalytisch wirksame Stoffe und/oder Verbindungen eingesetzt werden. Die vorgenannte Aufzählung ist nicht abschließend.

Die Luftreinigungseinheiten 5, 7 bestehen aus einem Rahmen und einem Vlies 51, 53, das den Rahmen umspannt, zumindest partiell, und einen Innenraum angrenzt, in dem Aktivkohle angeordnet oder der mit Aktivkohle versetzt ist. Dieser mit Aktivkohle durchsetzter bzw. versetzte bzw. versetzbare Bereich ist mit dem Bezugszeichen 52, 72 versehen.

Die Beleuchtungseinrichtung 6 ist auf die photokatalytisch wirksamen Bereiche 53, 73 abgestimmt. Die Beleuchtungseinheit 6 gibt UV-Licht oder sichtbares Licht mit einer voreinstellbaren oder voreingestellten Wellenlänge ab. Die Photonen mit der entsprechenden Wellenlänge des UV-Lichts oder sichtbaren Lichts der Beleuchtungseinrichtung 6 lösen beim Auftreffen auf den jeweiligen photokatalytisch wirksamen Bereich 53, 73 eine photochemischen Reaktion im Titanoxid aus, die dazu führt, dass Geruchsstoffpartikel und/oder Schadpartikel in der Luft umgewandelt bzw. zerstört werden. Die auf die photokatalytisch wirksamen Bereiche 53, 73 auftreffenden Geruchsstoffpartikel und/oder Schadpartikel werden durch den photochemischen Prozess zerstört oder umgewandelt und damit wird die zugeführte Luft gereinigt.

Bei der Beleuchtungseinrichtung 6 handelt es sich vorzugsweise um mindestens eine Leuchtdiode, vorzugsweise eine UV-Leuchtdiode. UV hat die Bedeutung von ultraviolett.

In einer vorteilhaften Ausgestaltung der Erfindung sind mehrere UV-Leuchtdioden angeordnet und bilden die Beleuchtungseinrichtung 6. Die Leuchtdioden sind zueinander in einer Reihe, äquidistant zueinander angeordnet. Es sind dann mehrere Reihen von UV-Leuchtdioden parallel nebeneinander und in gleichem Abstand zueinander anordenbar.

Die Beleuchtungseinrichtung 6 wird über eine nicht in FIG 1 dargestellten Steuereinheit angesteuert.

Im Gehäuse 2 sind Luftleitelemente 8 angeordnet. Diese dienen dazu, den Luftstrom 9 im Gehäuse 2 auf die Luftreinigungseinheiten 5, 7 und die photokatalytisch wirksamen Bereiche 53, 73 zu lenken, sodass ein möglichst großer Teil des Luftstroms 9 mit den Schadpartikeln und/oder Schadstoffpartikeln zu den photokatalytisch wirksamen Bereichen 53, 73 gelangt, um dort photokatalytisch zu reagieren. Um eine möglichst gute Luftumströmung der photokatalytisch wirksamen Bereiche 53, 73 der Luftreinigungseinheiten 5, 7 zu erreichen, und zugleich nicht die Lichtabgabe der Beleuchtungseinheit 6 auf die Luftreinigungseinheiten 5, 7 zu beeinträchtigen, sind die Luftleitelement 8 vor und nach der Beleuchtungseinheit 6 angeordnet. In einer vorteilhaften Ausgestaltung sind die Luftleitelemente 8 mit einer mit UV-Licht reflektierenden Beschichtung versehen.

Durch die Luftreinigungseinheiten 5, 7 und deren potokatalytisch wirksamen Bereiche 53, 73 wird, bei Auftreffen von UV-Licht, abgestrahlt von der Beleuchtungseinheit 6, entsprechend die das Gehäuse 2 durchströmende Luft gereinigt.

Jede Luftreinigungseinheit 5, 7 besitzt einen Rahmen, der von einem Vlies 51, 71 umspannt ist und damit einen Innenraum 52, 72 begrenzt. In den Innenraum 52, 72 ist Aktivkohle oder ein Granulat aus Aktivkohle und/oder ein Gemisch aus Aktivkohle und ein Granulat aus mit Titanoxid dotierten oder versetztem Granulat eingefüllt. Als Träger des Granulats für die Titanoxidionen kommen Eisenpartikel oder Kunststoffpartikel zur Anwendung. Auf der der Beleuchtungseinheit 6 zugeordneten Seite der Luftreinigungseinheit 5, 7 ist das dortig angeordnete Vlies 51, 71 mit einem fotokatalytisch wirksamen Oberflächenbereich 53, 73 ausgestattet. Der fotokatalytische und fotokatalytisch wirksame Oberflächenbereich 53, 73 ist mit Titanodxidionen versetzt bzw. durchsetzt. Durch auftreffen von UV-Licht oder Licht auf diese Bereiche 53, 73 wird der photokatalytische Prozess in Gang gesetzt. Schadstoffe, die mit dem Luftstrom 9 vorbeigeführt werden, werden entsprechend reduziert, zersetzt oder aufgelöst.

Das Vlies 51, 71 ist zumindest partiell, zur Ausbildung der photokatalytisch wirksamen Oberflächenbereiche 53, 73 mit Titanoxidionen dotiert oder versetzt oder aber es sind an diesen Stellen Fäden, oder Fasern, die mit Titanoxidionen dotiert oder versetzt sind, in das Vlies 51, 71 eingebracht, eingewebt oder mit diesem verbunden. Alternativ kann auch die Dotierung mit Titanoxidionen oder die Ausbildung dieser potokatalytisch wirksamen Bereiche 53, 57 erfolgen, in dem das Vlies 51, 71 mit einer Flüssigkeit, in der Titanoxidionen enthalten bzw. gelöst sind, getaucht wird oder mit dieser besprüht bzw. benetzt wird.

Das Vlies 51, 71 ist in der Weise ausgestaltet, dass es sogleich als Filter dient. Hierbei kann durchaus die Struktur eines HEPA-Filters als Vorlage für das Vlies 51, 71 dienen, sodass zusätzlich eine Reinigungswirkung von Grobpartikeln, die mit dem Luftstrom 9 angesaugt werden, erfolgt. Hierbei ist es vorteilhaft, dass das Vlies 51, 71 beidseitig an der Luftreinigungseinheit 5, 7 angeordnet ist, sodass bereits der eintretende Luftstrom 9 durch das Vlies 71 vorgereinigt wird und Grobpartikel aus dem Luftstrom 9 gefiltert werden.

Im Weiteren ist es vorteilhaft, die Oberfläche des Vlieses 51, 71 und damit die photokatalytisch wirksamen Bereiche 53, 73 großflächig auszugestalten. Daher hat es sich als vorteilhaft erwiesen die Oberfläche durch Faltung oder Verformung, beispielsweise durch Ausbildung einer Wellenform zu vergrößern. Im Weiteren ist es auch möglich diese Oberfläche mit kegel-, falten-, zylinder-, kegelstumpf-, pyramidenstumpf-, kugel-, oder halbkugelgeometrischen Ausformungen auszugestalten.

Ein weiterer Vorteil der Erfindung ist darin zu sehen, dass durch die photokatalytisch wirksamen Bereiche 53, 73 nicht nur die Luft, sondern auch die angrenzenden Bereiche mitgereinigt werden. Somit kann zugleich auch eine Reinigungswirkung auf das gesamte Vlies 51, 71, als auch auf die dahinter angeordnete Aktivkohle 52, 72 erfolgen.

Besonders vorteilhaft ist es, wie bereits ausgeführt, wenn das Titanoxid bzw. die Titanoxidionen auch in Form von Granulat oder in anderer Form in den Aktivkohlebereich 52, 72 direkt eingebunden werden. Dadurch wird, wenn die Titanoxidionen über den photokatalytischen Prozess aktiviert werden, auch die Aktivkohle selbst entsprechend gereinigt.

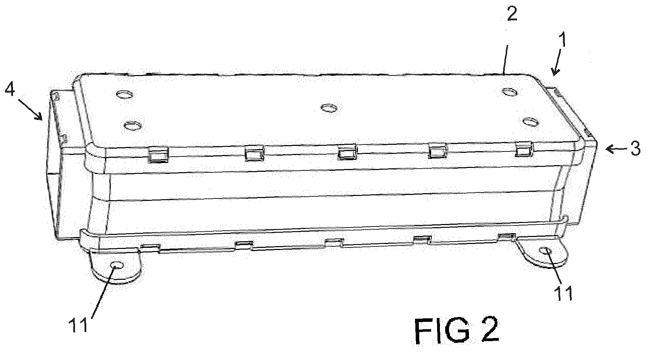

In FIG 2 ist das Gehäuse 2 der Luftreinigungsvorrichtung 1 perspektivisch dargestellt. Das Gehäuse 2 besteht aus mehreren Einzelteilen, die sich zu dem Gehäuse 2 zusammensetzen lassen. Das Gehäuse 2 ist quaderförmig ausgestaltet und weist zur einen Seite eine Verjüngung auf, die in den Lufteinlasskanal 3 übergeht. Auf der dem Lufteinlasskanal 3 gegenüberliegenden Seite des Gehäuses 2 ist eine weitere entsprechende Verjüngung vorgesehen, die die den Luftauslasskanal 4 bildet. Die zu reinigende Luft wird dem Kraftfahrzeuginnenraum entnommen, insbesondere aus diesem abgesaugt, und über den Lufteinlasskanal 3 dem Gehäuse 2 und damit der Luftreinigungsvorrichtung 1 zugeführt. Im Gehäuse 2 erfolgt dann die Reinigung der Luft, analog wie bei FIG 1 beschrieben, und die gereinigte Luft wird dann über den Luftauslasskanal 4 ausgeleitet und dann dem Fahrzeuginnenraum wieder zugeführt.

Im Weiteren sind am Gehäuse Aufnahmen 11 vorhanden, die fest mit dem Gehäuse 2 verbunden sind. Diese Aufnahmen 11 dienen dazu, die Luftreinigungsvorrichtung 1 in einem Kraftfahrzeug an eine vorgesehene Stelle befestigen zu können. Hierzu ist eine Klemmung oder eine Verschraubung vorgesehen.

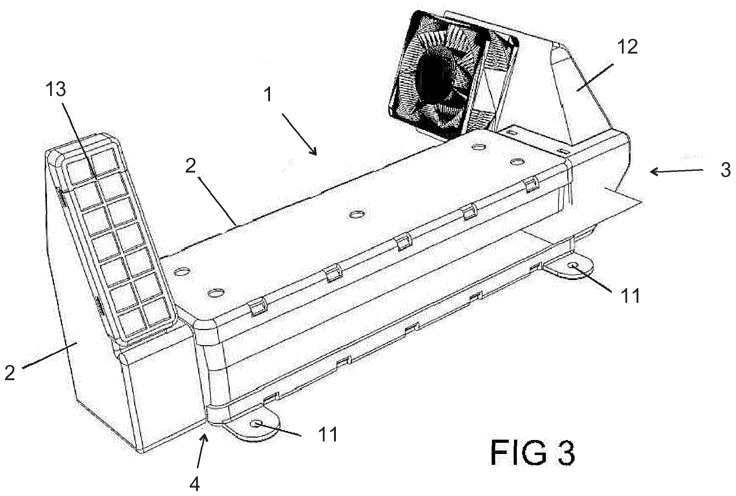

In FIG 3 ist die Luftreinigungsvorrichtung 1 mit dem Gehäuse 2 dargestellt. Es ist am Lufteinlasskanal 3 ein Luftzuführungskanal 12 angeordnet, an dessen Eingang ein Ventilator 11 angeordnet ist, der Luft aus dem Fahrzeuginnenraum ansaugt und durch den Luftzuführungskanal 12 und über den Lufteinlasskanal 3 in das Gehäuse 2 der Luftreinigungsvorrichtung 1 bläst, vorhanden. Der Luftzuführungskanal 12 ist auf die Verjüngung des Lufteinlasskanals 3 aufgesteckt und ist formschlüssig mit diesem verbunden.

In einer besonderen Ausgestaltung der Erfindung ist der Luftzuführungskanal 12 mittels einer Schnappvorrichtung mit dem Lufteinlasskanal 3 oder dem Gehäuse 2 verbindbar. Auf der Seite der Luftausgangsöffnung 4 ist ein Luftführungskanal 13 angeordnet, der die ausströmende Luft aus der Luftausgangsöffnung 4 aufnimmt, kanalisiert und entsprechend an den Innenraum eines Kraftfahrzeuges, aus dem mittels des Ventilators 11 die Luft abgesaugt worden ist, wieder zugeführt.

In einer weiteren vorteilhaften Ausgestaltung der Erfindung ist vorgesehen, dass in dem Luftführungskanal 12 mindestens eine weitere Luftfiltereinheiten, wie beispielsweise ein HEPA-Filter oder ein anderer Luftfilter, angeordnet ist.

Das Gehäuse 2 besitzt einen Gehäuseboden und einen Gehäusedeckel, die gemeinsam das Gehäuse 2 bilden. Am Gehäuseboden sind die Aufnahmen 11 angeordnet. Seitlich außenseitig weisen sowohl der Gehäuseboden als auch der Gehäusedeckel, mehrere Einbuchtungen auf, in welche korrespondierende Nasen an den Gehäuseseitenteilen einschnappen und so den Gehäuseboden und den Gehäusedeckel über die Gehäuseseitenteile verbinden.

Auf der Innenseite des Gehäusedeckels und der Innenseite des Gehäusebodens ist die Luftreinigungseinheit 5, 7 angeordnet bzw. dort fixiert. Jede Luftreinigungseinheit 5, 7 ist form- und/oder kraftschlüssig mit dem Gehäuseboden oder dem Gehäusedeckel verbunden. Diese Verbindung erfolgt beispielsweise über eine Verklipsung, Verklebung oder Verschraubung.

Bei der Beleuchtungseinheit 6 handelt es sich um eine oder mehrere Leuchtdioden, die ultraviolettes Licht emittieren, das vorzugsweise auf das Titandioxid und dessen dadurch erzeugbaren photokatalytischen Wirkung abgestimmt ist, und eine Wellenlänge im Bereich zwischen 250 und 400 Nanometer besitzt, vorzugsweise im Bereich um die 367 Nanometer. Bei Einsatz von dotiertem TiO2 kann sichtbares Licht im Bereich von 400 bis 500 Nanometer zum Einsatz kommen, vorzugsweise mit 455 nm Wellenlänge.

### Bezugszeichenliste

- 1: Luftreinigungsvorrichtung
- 2: Gehäuse
- 3: Lufteinlasskanal
- 4: Luftauslasskanal
- 5, 7: Luftreinigungseinheit
- 51, 71: Vlies
- 52, 72: mit Aktivkohle durchsetzter Bereich
- 53, 73: photokatalytisch wirksamer Oberflächenbereich
- 6, 26: Beleuchtungseinheit
- 8: Luftleitelemente
- 9: Luftstrom
- 10: Ventilator
- 11: Aufnahme(n)
- 12: Luftzuführungskanal
- 13: Luftführungskanal

## Patentansprüche

1. Luftreinigungsvorrichtung (1), bestehend aus einem Gehäuse (2), das mindestens eine Eingangsöffnung (3) zur Zuführung eines Luftstroms (9) und mindestens eine Ausgangsöffnung (4) zur Ausleitung des über die Eingangsöffnung (3) zugeführten Luftstroms (9) besitzt, wobei im Gehäuse (2) mindestens eine Luftreinigungseinheit (5, 7) und mindestens eine Beleuchtungseinheit (6) angeordnet sind, wobei die mindestens eine Luftreinigungseinheit (5, 7) im Wesentlichen die Form des Querschnitts des Gehäuses (2) aufnimmt und die Querschnittsfläche nahezu ausfüllend im Gehäuse (2) angeordnet ist, im Gehäuse (2) die mindestens eine Beleuchtungseinheit (6) der mindestens eine Luftreinigungseinheit (5, 7) gegenüberliegend angeordnet ist, die mindestens eine Luftreinigungseinheit (5, 7) luftdurchlässig ausgestaltet ist und mindestens einen mit Aktivkohle durchsetzten Bereich (52, 72) aufweist, der zumindest partiell von einem Vlies (51, 71) umgeben ist,
wobei auf der der Beleuchtungseinheit (6) zugewandten Seite der mindestens einen Luftreinigungseinheit (5, 7) mindestens ein photokatalytisch wirksamer Oberflächenbereich (53, 73) im oder am Vlies (51, 71) vorhanden ist, wozu zumindest partiell Fäden oder Fasern in das Vlies (51, 71) eingearbeitet oder eingebracht sind, die mit Titandioxidionen - TiO2-Ionen dotiert sind, welche den mindestens einen photokatalytisch wirksamen Oberflächenbereich (53, 73) ausbildet oder dass Bereiche (53, 73) des Vlieses (51, 71) mit Titandioxidionen - TiO2-Ionen dotiert sind, und wobei der Luftstrom (9) im Gehäuse (2) zumindest partiell den mindestens einen photokatalytisch wirksamen Oberflächenbereich (53, 73) und den mit Aktivkohle durchsetzen Flächenbereich (52, 72) umströmt bzw. durchströmt, wobei der mindestens eine photokatalytisch wirksame Oberflächenbereich (53, 73) der mindestens einen Luftreinigungseinheit (5, 7) von der mindestens einen Beleuchtungseinheit (6) mit Licht anstrahlbar ist.

2. Luftreinigungsvorrichtung (1) nach Patentanspruch 1, **dadurch gekennzeichnet, dass** der mindestens eine photokatalytisch wirksame Oberflächenbereich (53, 73) zumindest teilweise mit einem Metalloxid oder einem Mischoxid beschichtet oder mit einem Metalloxid oder einem Mischoxid - versetzt oder durchsetzt ist.

3. Luftreinigungsvorrichtung (1) nach Patentanspruch 2, **dadurch gekennzeichnet, dass** das Metalloxid ein Oxid aus einem Übergangsmetall ist.

4. Luftreinigungsvorrichtung (1) nach einem der vorangehenden Patentansprüche 2 oder 3, **dadurch gekennzeichnet, dass** das Metalloxid oder Mischoxid aus CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ und/oder Nb₂O₅ besteht.

5. Luftreinigungsvorrichtung (1) nach Patentanspruch 1, **dadurch gekennzeichnet, dass** die mindestens eine Luftreinigungseinheit (5, 7) an einer der Innenwände des Gehäuses (2) fixierbar ist.

6. Luftreinigungsvorrichtung (1) nach einem der vorangehenden Patentansprüche 1 bis 5, **dadurch gekennzeichnet, dass** die mindestens eine Beleuchtungseinheit (6) im Gehäuse (2) der mindestens einen Luftreinigungseinheit (5, 7) gegenüberliegend zum mindestens einen photokatalytisch wirksamen Oberflächenbereich (53, 73) der mindestens einen Luftreinigungseinheit (5, 7) auf einer der Innenwände des Gehäuses (2) oder nahezu in der Mitte des Gehäuses (2) angeordnet ist, und wobei die Innenwände des Gehäuses (2) im Bereich zwischen der mindestens einen Luftreinigungseinheit (5, 7) und der mindestens eine Beleuchtungseinheit (6) mit einem lichtreflecktierenden Material, insbesondere einem UV-Licht reflecktierenden Material, beschichtet sind.

7. Luftreinigungsvorrichtung (1) nach einem der vorangehenden Patentansprüche, **dadurch gekennzeichnet, dass** vor der mindestens einen Beleuchtungseinheit (6) Linsen angeordnet sind, die das von der mindestens einen Beleuchtungseinheit (6) emittierte Licht auf die mindestens eine Luftreinigungseinheit (5, 7) fokussieren oder hinter und/oder neben der mindestens einen Beleuchtungseinheit (6) Lichtreflektoren oder Licht reflektierende Bereiche angeordnet sind, welche das von der mindestens einen Beleuchtungseinheit (6) emittierte Licht in Richtung der mindestens einen Luftreinigungseinheit (5, 7) reflektieren.

8. Luftreinigungsvorrichtung (1) nach einem der vorangehenden Patentansprüche, **dadurch gekennzeichnet, dass** die Innenwände des Gehäuses (2, 22) weiß lackiert oder mit einem lichtreflektierenden Material, insbesondere einem UV-Licht reflektierden Material, beschichtet sind.

9. Luftreinigungsvorrichtung (1) nach einem der vorangehenden Patentansprüche, **dadurch gekennzeichnet, dass** im Gehäuse (2) Luftleitelemente (8) angeordnet sind, welche den Luftstrom (9) diffus im Gehäuse (2) ablenken, sodass der Luftstrom (9) nahezu gleichmäßig auf die gesamte Oberfläche des Vlieses (51, 71) der mindestens einen Luftreinigungseinheit (5, 7) trifft und diese durchströmt oder der Luftstrom (9) größtenteils auf den mindestens einen photokatalytisch wirksamen Oberflächenbereich (53, 73) gerichtet ist und diesen umströmt bzw. durchströmt.

10. Luftreinigungsvorrichtung (1) nach einem der vorangehenden Patentansprüche, **dadurch gekennzeichnet, dass** die mindestens eine Luftreinigungseinheit (5, 7) aus einem Rahmen aus Kunststoff oder Metall besteht, welcher vom Vlies (51, 71) zumindest an einer Seite umschlossen ist und hinter dem Vlies (51, 71) ein Aufnahmebereich für Granulatstoffe vorhanden ist, wobei dieser Aufnahmebereich mit Aktivkohle oder einem Gemisch aus Aktivkohle und mit Titandioxid - TiO2 - beschichtetem oder mit Titandioxidionen - TiO2-Ionen - versetztem Granulat befüllt ist.

11. Luftreinigungsvorrichtung (1) nach einem der vorangehenden Patentansprüche, **dadurch gekennzeichnet, dass** die Oberfläche der mindestens einen Luftreinigungseinheit (5, 7) mit dem Vlies (51, 71) zumindest an der den mindestens einen photokatalytisch wirksamen Oberflächenbereich (53, 73) aufweisenden Seite wellenförmig ausgestaltet ist oder kegel-, falten-, zylinder-, kegelstumpf-, pyramidenstumpf-, kugel- oder halbkugelgeometrische Ausformungen besitzt.

12. Luftreinigungsvorrichtung (1) nach einem der vorangehenden Patentansprüche, **dadurch gekennzeichnet, dass** die mindestens eine Beleuchtungseinheit (6) in ihrer Lichtintensität steuerbar ist, wobei ein Luftgütemesssensor der im Lufteinlasskanal (3) oder am Luftauslasskanal (4) angeordnet ist die Luftgüte ermittelt und eine Steuereinheit anhand der vom Luftgütemesssensor ermittelten Luftgüte die Lichtintensität der mindestens eine Beleuchtungseinheit (6) steuert.

13. Luftreinigungsvorrichtung (1) nach einem der vorangehenden Patentansprüche, **dadurch gekennzeichnet, dass** die mindestens eine Beleuchtungseinheit (6) in ihrer Lichtintensität von der Steuereinheit derart steuerbar ist, dass die Steuereinheit die Lichtintensität der mindestens eine Beleuchtungseinheit (6) in Abhängigkeit der Drehzahl eines Ventilators steuert, der vor dem Lufteinlasskanal (3) oder dem Luftauslasskanal (4) angeordnet ist.

14. Luftreinigungsvorrichtung (1) nach einem der vorangehenden Patentansprüche, **dadurch gekennzeichnet, dass** die Luftreinigungsvorrichtung (1) einen VOC Sensors zur bedarfsgerechten Steuerung der Luftreinigungsvorrichtung (1) aufweist.

## Claims

1. Air purifying device (1) consisting of a housing (2) having at least one inlet opening (3) for supply of an air flow (9) and at least one outlet opening (4) for conducting out the air flow (9) supplied by way of the inlet opening (3), wherein at least one air purifying unit (5, 7) and at least one lighting unit (6) are arranged in the housing (2), wherein the at least one air purifying unit (5, 7) takes on substantially the form of the cross-section of the housing (2) and is arranged in the housing (2) to almost fill out the cross-sectional area, the at least one lighting unit (6) is arranged in the housing (2) to be opposite the at least one air purifying unit (5, 7), and the at least one air purifying unit (5, 7) is formed to be permeable to air and has at least one region (52, 72) which is permeated with activated carbon and which is surrounded at least partly by a non-woven material (51, 71), wherein at least one photocatalytically active surface region (53, 73) in or at the non-woven material (51, 71) is present on the side of the at least one air purifying unit (5, 7) facing the lighting unit (6), for which purpose threads or fibres doped with titanium dioxide ions (TiO₂ ions) are worked or introduced into the non-woven material (51, 71) at least in part, which forms the at least one photocatalytically active surface region (53, 73), or regions (53, 73) of the non-woven material (51, 71) are doped with titanium dioxide ions (TiO₂ ions), and wherein the air flow (9) in the housing (2) at least in part flows around or through the at least one photocatalytically active surface region (53, 73) and the surface region (52, 72) permeated by activated carbon, wherein the at least one photocatalytically active surface region (53, 73) of the at least one air purifying unit (5, 7) can be irradiated with light by the at least one lighting unit (6).

2. Air purifying device (1) according to claim 1, **characterised in that** the at least one photocatalytically active surface region (53, 73) is coated at least partly with a metal oxide or a mixed oxide or is packed or permeated with a metal oxide or a mixed oxide.

3. Air purifying device (1) according to claim 2, **characterised in that** the metal oxide is an oxide of a transition metal.

4. Air purifying device (1) according to one of the preceding claims 2 and 3, **characterised in that** the metal oxide or mixed oxide is of CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ and/or Nb₂O₅.

5. Air purifying device (1) according to claim 1, **characterised in that** the at least one air purifying unit (5, 7) is fixable to one of the inner walls of the housing (2).

6. Air purifying device (1) according to any one of the preceding claims 1 to 5, **characterised in that** the at least one lighting unit (6) is arranged in the housing (2) of the at least one air purifying unit (5, 7) to be opposite the at least one photocatalytically active surface region (53, 73) of the at least one air purifying unit (5, 7) on one of the inner walls of the housing (2) or approximately in the middle of the housing (2), wherein the inner walls of the housing (2) are coated in the region between the at least one air purifying unit (5, 7) and the at least one lighting unit (6) with a light-reflective material, particularly an ultraviolet-light-reflective material.

7. Air purifying device (1) according to any one of the preceding claims, **characterised in that** lenses which focus the light emitted by the at least one lighting unit (6) onto the at least one air purifying unit (5, 7) are arranged in front of the at least one lighting unit (6) or light reflectors or light-reflective regions are arranged behind and/or adjacent to the at least one lighting unit (6) and reflect the light, which is emitted by the at least one lighting unit (6), in the direction of the at least one air purifying unit (5, 7).

8. Air purifying device (1) according to any one of the preceding claims, **characterised in that** the inner walls of the housing (2, 22) are painted white or coated with a light-reflective material, particularly an ultraviolet-light-reflective material.

9. Air purifying device (1) according to any one of the preceding claims, **characterised in that** air guide elements (8) deflecting the air flow (9) diffusely in the housing (2) are arranged in the housing (2) so that the air flow (9) impinges almost uniformly on the entire surface of the non-woven material (51, 71) of the at least one air purifying unit (5, 7) and flows therethrough or the air flow (9) for the major part is directed onto the at least one photocatalytically active surface region (53, 73) and flows therearound or therethrough.

10. Air purifying device (1) according to any one of the preceding claims, **characterised in that** the at least one air purifying unit (5, 7) consists of a frame of plastics material or metal which is enclosed by the non-woven material (51, 71) at least at one side and a receiving region for granulate materials is present behind the non-woven material (51, 71), wherein this receiving region is filled with activated carbon or a mixture of activated carbon and granulate coated with titanium dioxide (TiO₂) or packed with titanium dioxide ions (TiO₂ ions).

11. Air purifying device (1) according to any one of the preceding claims,
**characterised in that** the surface of the at least one air purifying unit (5, 7) with the nonwoven material (51, 71) at least at the side having the at least one photocatalytically active surface region (53, 73) is formed to be wave-shaped or has conical, folded, cylindrical, frusto-conical, frusto-pyramidal, spherical or hemispherical geometric shaping.

12. Air purifying device (1) according to any one of the preceding claims,
**characterised in that** the at least one lighting unit (6) is controllable in the light intensity thereof, wherein an air quality measuring sensor arranged in the air inlet channel (3) or
at the air outlet channel (4) determines the air quality and a control unit controls the light intensity of the at least one lighting unit (6) on the basis of the air quality determined by the air quality measuring sensor.

13. Air purifying device (1) according to any one of the preceding claims,
**characterised in that** the at least one lighting unit (6) is so controllable in the light intensity thereof by the control unit that the control unit controls the light intensity of the at least one lighting unit (6) in dependence on the rotational speed of a fan arranged upstream of the air inlet channel (3) or the air outlet channel (4).

14. Air purifying device (1) according to any one of the preceding claims,
**characterised in that** the air purifying device (1) comprises a volatile organic compound sensor for control of the air purifying device (1) appropriately to requirements.

## Revendications

1. Dispositif d'épuration de l'air (1), consistant en un boîtier (2), qui possède au moins une ouverture d'entrée (3) pour introduire un courant d'air (9) et au moins une ouverture de sortie (4) pour évacuer le courant d'air (9) introduit par l'ouverture d'entrée (3), où au moins une unité d'épuration de l'air (5, 7) et au moins une unité d'éclairage (6) sont disposées dans le boîtier (2), où la au moins une unité d'épuration de l'air (5, 7) revêt sensiblement la forme de la section droite du boîtier (2) et est disposée dans le boîtier (2) en remplissant sensiblement la surface de la section droite, dans le boîtier (2) la au moins une unité d'éclairage (6) est disposée en face de la au moins une unité d'épuration de l'air (5, 7), la au moins une unité d'épuration de l'air (5, 7) est agencée de manière perméable à l'air et présente au moins un domaine chargé de charbon actif (52, 72) qui est entouré au moins en partie par un feutre (51, 71), où au moins un domaine de surface efficace du point de vue photocatalytique (53, 73) est présent dans ou sur le feutre (51, 71) sur le côté de la au moins une unité d'épuration de l'air (5, 7) tourné vers l'unité d'éclairage (6), ce pour quoi des fils ou des fibres sont incorporés ou introduits au moins en partie dans le feutre (51, 71), qui dont dopés avec des ions dioxyde de titane - ions TiO₂, qui forment le au moins un domaine de surface efficace du point de vue photocatalytique (53, 73) ou en ce que des domaines (53, 73) du feutre (51, 71) sont dopés avec des ions dioxyde de titane - ions TiO₂, et où le courant d'air (9) dans le boîtier (2) s'écoule autour de ou traverse au moins en partie le au moins un domaine de surface efficace du point de vue photocatalytique (53, 73) et le domaine de surface chargé de charbon actif (52, 72), où le au moins un domaine de surface efficace du point de vue photocatalytique (53, 73) de la au moins une unité d'épuration de l'air (5, 7) peut être irradié avec de la lumière par la au moins une unité d'éclairage (6).

2. Dispositif d'épuration de l'air (1) selon la revendication 1, **caractérisé en ce que** le au moins un domaine de surface efficace du point de vue photocatalytique (53, 73) est revêtu au moins en partie d'un oxyde métallique ou d'un oxyde mixte ou est additionné de ou chargé avec un oxyde métallique ou un oxyde mixte.

3. Dispositif d'épuration de l'air (1) selon la revendication 2, **caractérisé en ce que** l'oxyde métallique est un oxyde d'un métal de transition.

4. Dispositif d'épuration de l'air (1) selon l'une des revendications 2 ou 3 précédentes, **caractérisé en ce que** l'oxyde métallique ou l'oxyde mixte de CuO, Co₃O₄, CoOₓ, NiO, MnOₓ, MnO₂, MoO₃, ZnO, Fe₂O₃, WO₃, CeO₂, TiO₂, Al₂O₃, V₂O₃, ZrO₂, HfO₂, Dy₂O₃, Cr₂O₃ et/ou Nb₂O₅.

5. Dispositif d'épuration de l'air (1) selon la revendication 1, **caractérisé en ce que** la au moins une unité d'épuration de l'air (5, 7) peut être fixée sur l'une des parois internes du boîtier (2).

6. Dispositif d'épuration de l'air (1) selon l'une des revendications 1 à 5 précédentes, **caractérisé en ce que** la au moins une unité d'éclairage (6) est disposée dans le boîtier (2) en face de la au moins une unité d'épuration de l'air (5, 7) vers au moins un domaine de surface efficace du point de vue photocatalytique (53, 73) de la au moins une unité d'épuration de l'air (5, 7) sur l'une des parois internes du boîtier (2) ou sensiblement au milieu du boîtier (2), et où les parois internes du boîtier (2) dans le domaine entre la au moins une unité d'épuration de l'air (5, 7) et la au moins une unité d'éclairage (6) sont revêtues d'un matériau réfléchissant la lumière, en particulier d'un matériau réfléchissant la lumière UV.

7. Dispositif d'épuration de l'air (1) selon l'une des revendications précédentes, **caractérisé en ce que** devant la au moins une unité d'éclairage (6) sont disposées des lentilles qui focalisent sur la au moins une unité d'épuration de l'air (5, 7) la lumière émise par la au moins une unité d'éclairage (6) ou derrière et/ou à côté de la au moins une unité d'éclairage (6) sont disposés des réflecteurs de lumière ou des domaines réfléchissant la lumière qui réfléchit la lumière émise par la au moins une unité d'éclairage (6) en direction de la au moins une unité d'épuration de l'air (5, 7).

8. Dispositif d'épuration de l'air (1) selon l'une des revendications précédentes, **caractérisé en ce que** les parois internes du boîtier (2, 22) sont peintes en blanc ou sont revêtues d'un matériau réfléchissant la lumière, en particulier d'un matériau réfléchissant la lumière UV.

9. Dispositif d'épuration de l'air (1) selon l'une des revendications précédentes, **caractérisé en ce que** dans le boîtier (2) sont disposés des éléments guidant l'air (8) qui dévient le courant d'air (9) de manière diffuse dans le boîtier (2), de sorte que le courant d'air (9) parvient de manière sensiblement uniforme sur toute la surface du feutre (51, 71) de la au moins une unité d'épuration de l'air (5, 7) et traverse celle-ci ou le courant d'air (9) est dirigé pour la plus grande part sur le au moins un domaine de surface efficace du point de vue photocatalytique (53, 73) et s'écoule autour de celui-ci ou traverse celui-ci.

10. Dispositif d'épuration de l'air (1) selon l'une des revendications précédentes, **caractérisé en ce que** la au moins une unité d'épuration de l'air (5, 7) consiste en un cadre en matière synthétique ou en métal qui est entouré par le feutre (51, 71) au moins d'un côté et un domaine de logement pour des substances granulées est présent derrière le feutre (51, 71), où ce domaine de logement est rempli de charbon actif ou d'un mélange de charbon actif et de granulés revêtus de dioxyde de titane - TiO2 - ou chargés d'ions dioxyde de titane - ions TiO2.

11. Dispositif d'épuration de l'air (1) selon l'une des revendications précédentes, **caractérisé en ce que** la surface de la au moins une unité d'épuration de l'air (5, 7) est agencée de manière ondulée avec le feutre (51, 71) au moins du côté présentant le au moins un domaine de surface efficace du point de vue photocatalytique (53, 73) ou possède des éléments géométriques coniques, plissés, cylindriques, tronconiques, en pyramide tronquée, sphériques ou hémisphériques.

12. Dispositif d'épuration de l'air (1) selon l'une des revendications précédentes, **caractérisé en ce que** la au moins une unité d'éclairage (6) peut être commandée concernant son intensité lumineuse, où un capteur de mesure de la qualité de l'air qui est disposé dans le canal d'entrée d'air (3) ou sur le canal de sortie d'air (4) détermine la qualité de l'air et une unité de commande commande l'intensité lumineuse de la au moins une unité d'éclairage (6) à l'aide de la qualité de l'air déterminée par le capteur de mesure de la qualité de l'air.

13. Dispositif d'épuration de l'air (1) selon l'une des revendications précédentes, **caractérisé en ce que** la au moins une unité d'éclairage (6) peut être commandée concernant son intensité lumineuse par l'unité de commande de telle manière que l'unité de commande commande l'intensité lumineuse de la au moins une unité d'éclairage (6) en fonction de la vitesse de rotation d'un ventilateur qui est disposé devant le canal d'entrée d'air (3) ou le canal de sortie d'air (4).

14. Dispositif d'épuration de l'air (1) selon l'une des revendications précédentes, **caractérisé en ce que** le dispositif d'épuration de l'air (1) présente un capteur de COV pour la commande conforme aux besoins du dispositif d'épuration de l'air (1).
